# FASCICULE DE BREVET EUROPEEN

(11) **EP 0 926 991 B1**
(45) Date de publication et mention de la délivrance du brevet: **26.06.2002**
(21) Numéro de dépôt: 97935644.1
(22) Date de dépôt: 29.07.1997
(51) Int. Cl.: A61B 17/122

(54) **SUPPORT DE CLIPS HEMOSTATIQUES**
TRÄGER FÜR HÄMOSTATISCHE KLAMMERN
HAEMOSTATIC CLIP HOLDER

(30) Priorité: 01.08.1996 FR 9609921
(43) Date de publication de la demande: 07.07.1999
(73) Titulaire: Vitalitec International, 35500 Vitre (FR)
(72) Inventeur: FORSTER, Michel, F-26760 Beaumont-lès-Valence (FR)
(74) Mandataire: Le Faou, Daniel
(86) Numéro de dépôt international: FR9701410
(87) Numéro de publication internationale: WO9805260

(56) Documents cités:
- EP-A- 0 482 861
- EP-A- 0 583 151
- WO-A-91/04925
- US-A- 4 076 120
- US-A- 4 294 355
- US-A- 4 961 499

## Description

La présente invention concerne un support de clips hémostatiques.

Un clip hémostatique est une petite agrafe, généralement métallique, ayant la forme approximative d'un "U", qui est destinée à être placée et refermée sur un vaisseau sanguin au cours d'une intervention chirurgicale, pour obturer ledit vaisseau.

Les clips sont fournis aux utilisateurs sur un support, usuellement appelé cartouche ou barrette, qui contient un certain nombre de clips placés côte-à-côte, par exemple une dizaine.

Au cours de son intervention, le chirurgien prélève les clips un par un, au fur et à mesure de ses besoins, à l'aide d'une pince spéciale qui lui sert également, après prélèvement, à transférer le clip sur le vaisseau, et à l'écraser pour pincer et obturer ce dernier.

Les supports de clips comprennent usuellement un certain nombre de logements disposés transversalement par rapport à la direction longitudinale de la barrette, dans lesquels les clips sont emboîtés, en position renversée ("U" à l'envers).

Des dispositifs de ce genre sont par exemple décrits dans les documents US-A-3 326 216 et 4 696 396.

Dans ces dispositifs connus, la retenue du clip est réalisée par friction, les branches du "U" étant précontraintes dans le sens de la fermeture, de manière à enserrer la paroi de barrette avec un certain frottement.

Cet effort de maintien est assez aléatoire, et l'extraction des clips au moyen de la pince n'est pas toujours facile, de sorte que le support de clips a tendance à se soulever avec la pince d'extraction.

Il est donc souvent nécessaire de faire usage des deux mains, ce qui n'est pas pratique pour le chirurgien.

On connaît par ailleurs, par le document WO-91/04925, un support de clips du genre mentionné, dans lequel la retenue des clips est réalisée au moyen de languettes élastiques à encoches, qui coopèrent avec les branches latérales du clip renversé.

Si ce dispositif permet en principe d'éliminer l'inconvénient qui vient d'être mentionné, il se pose néanmoins un problème d'interférence de la pince avec les languettes de maintien, puisque celles-ci sont en appui contre les branches du clip, parties contre lesquelles se fait également l'engagement de la pince.

Ceci est cause de dysfonctionnements. De plus, la présence de languette déformable latérale, de chaque côté du clip, accroît très sensiblement l'encombrement en largeur de la barrette.

Dans le dispositif qui fait l'objet du document US-A-4 961 499, chaque clip est emboîté à califourchon sur un élément fixe, de forme complémentaire, bordé par une paire de parois transversales massives servant à retenir le clip. Pour cela, ces parois présentent, en regard l'une de l'autre, des protubérances aptes à bloquer la zone centrale du fond du clip. Les parois possèdent une certaine souplesse due à la présence de fentes ménagées dans leur épaisseur et elles peuvent s'écarter temporairement l'une de l'autre pour permettre le passage du clip, par effet de rampe, au moment de sa mise en place et de son enlèvement.

Malgré cela, la raideur des parois est relativement élevée, et ce dispositif ne permet pas d'extraire les clips en développant un effort réduit et constant ; au contraire, l'opérateur rencontre un point dur, et il n'est pratiquement pas possible de faire usage d'une pièce à rainures débouchantes pour procéder à l'extraction. En effet, avec ce type de pince, la liaison entre les mors de la pince et le clip se fait uniquement par friction, et elle ne peut pas vaincre un effort antagoniste élevé et/ou brutal.

Les mêmes inconvénients se rencontrent avec les dispositifs décrits dans les documents US-A-4 076 120 et EP-A-0 482 861 qui, comme le précédent, réalisent le blocage de la zone centrale du fond du clip par deux éléments qui se font face, symétriquement de part et d'autre du plan du clip, et ne peuvent se déformer que sur une course très limitée.

Le dispositif du document EP-A-0 583 151 - qui est une évolution du EP-A-0 482 861- s'adresse à un clip de forme particulière, non symétrique. Il possède une petite languette élastique, en forme de crochet, agissant latéralement contre le clip. Ce dispositif présente une configuration qui confère à la languette une course élastique également très limitée ; de plus, elle n'est pas adaptée pour supporter et retenir des clips en forme de "U".

La présente invention vise à résoudre ces problèmes en proposant un support de clips hémostatiques du genre mentionné, destiné à recevoir des clips en forme générale et approximative de "U", qui soit d'une conception simple, qui assure une retenue correcte de l'ensemble des clips, tout en étant facile d'utilisation, aussi bien lors de la pose des clips en usine que lors de leur extraction par le chirurgien, au moment de l'intervention, cette extraction pouvant se faire en développant un effort relativement faible et sensiblement constant, même à l'aide d'une pince à rainures débouchantes.

Ce support de clips s'adresse à un ensemble de clips ayant approximativement la forme d'un "U" comprenant une partie de fond et une paire de branches latérales, et il consiste en une barrette allongée présentant des moyens pour supporter et retenir individuellement les clips côte-à-côte en position renversée, dans des plans parallèles transversaux, ces moyens coopérant exclusivement avec les parties de fond du clip, tandis que ses branches latérales sont libres.

Ce support est remarquable par le fait que lesdits moyens de support et de retenue de chacun des clips consistent, d'une part, en une partie fixe qui porte au moins une face d'appui sur laquelle vient reposer la partie de fond du clip, d'autre part, en une partie mobile en forme de languette mince et souple, élastiquement déformable par flexion, dont la longueur est au moins égale - et de préférence supérieure - à la hauteur du clip, et qui comporte en partie haute une patte de blocage à paroi convexe (bombée) venant s'appliquer contre la zone centrale de ladite partie de fond du clip pour la retenir, ces deux parties présentant des parois d'entrée convergentes qui assurent le guidage du clip et l'écartement provisoire de la patte de blocage au moyen de sa mise en place.

Ainsi, dans le support qui fait l'objet de l'invention, les moyens qui assurent le support et le blocage de chaque clip n'ont pas une configuration symétrique par rapport au plan contenant le clip. Au contraire, ils sont dissociés, les moyens (fixes) réalisant l'appui du clip étant situés d'un côté, tandis que les moyens (mobiles) réalisant son blocage sont situés de l'autre côté.

Du fait que la languette est mince et longue, elle possède une très grande flexibilité (faible raideur) autorisant le prélèvement et l'extraction "en douceur" du clip sous un effort faible et pratiquement constant au moyen d'une pince, y compris une pince dont les extrémités des rainures sont ouvertes.

La languette souple peut fléchir en s'écartant de la partie fixe, suivant une trajectoire perpendiculaire au plan du clip.

Par ailleurs, selon un certain nombre de caractéristiques additionnelles, non limitatives de l'invention :
- la partie fixe possède deux faces d'appui qui supportent les zones intermédiaires du clip situées entre la zone centrale et les branches latérales ;
- la partie fixe possède une face formant butée de fin de course pour la pince de prélèvement des clips ;
- les différentes parties fixes et déformables sont placées tête-bêche, deux à deux, de sorte que chaque partie fixe - à l'exception des parties d'extrémité - assure la retenue de deux clips, en association avec deux pattes déformables.

Cette disposition permet d'accroître la compacité du dispositif.

Dans un mode de réalisation possible, plus particulièrement adapté aux clips de taille moyenne ou faible, la partie mobile en forme de languette possède une largeur sensiblement inférieure à celle du clip, et elle est bordée d'éléments fixes en regard desquels viennent se placer les branches latérales du clip.

Grâce à cette disposition, la mise en place incorrecte de la pince ne risque pas de solliciter prématurément la languette, en provoquant une extraction intempestive du clip, car les branches de la pince viennent se positionner obligatoirement en regard des éléments fixes.

D'autres caractéristiques et avantages de l'invention apparaîtront de la description qui va maintenant être faite d'un mode de réalisation préféré, en référence aux dessins annexés sur lesquels :
- la figure 1 est une vue de face du support, partiellement coupé par un plan vertical longitudinal ;
- la figure 2 est une vue de dessus du support de la figure 1, partiellement coupée par un plan horizontal ;
- les figures 3 et 4 sont des vues en coupe transversale, respectivement selon les plans référencés III-III et IV-IV sur la figure 1 ;
- la figure 5 est une vue schématique en coupe transversale, qui illustre la manière dont se fait le prélèvement d'un clip au moyen d'une pince ad'hoc ;
- la figure 6 représente un clip ;
- la figure 7 est un schéma à grande échelle montrant la manière dont se fait le pincement du clip sur le support au moment de son engagement sur celui-ci (mise en place en usine).
   Les figures 8 à 13 représentent une variante du support.

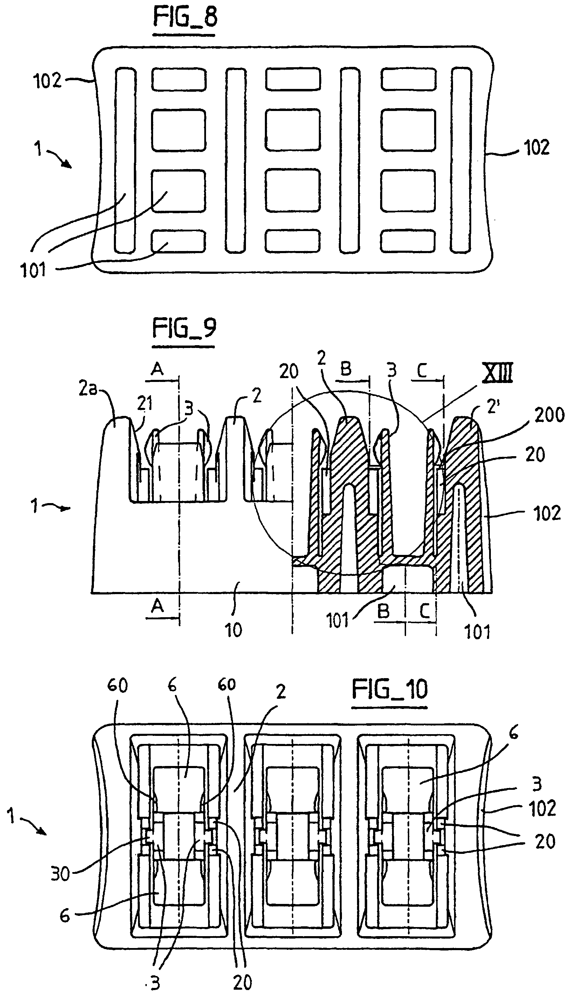

- les figures 8, 9, et 10 sont des vues de dessous, de face et partiellement coupée et, respectivement, de dessus de cette variante ;

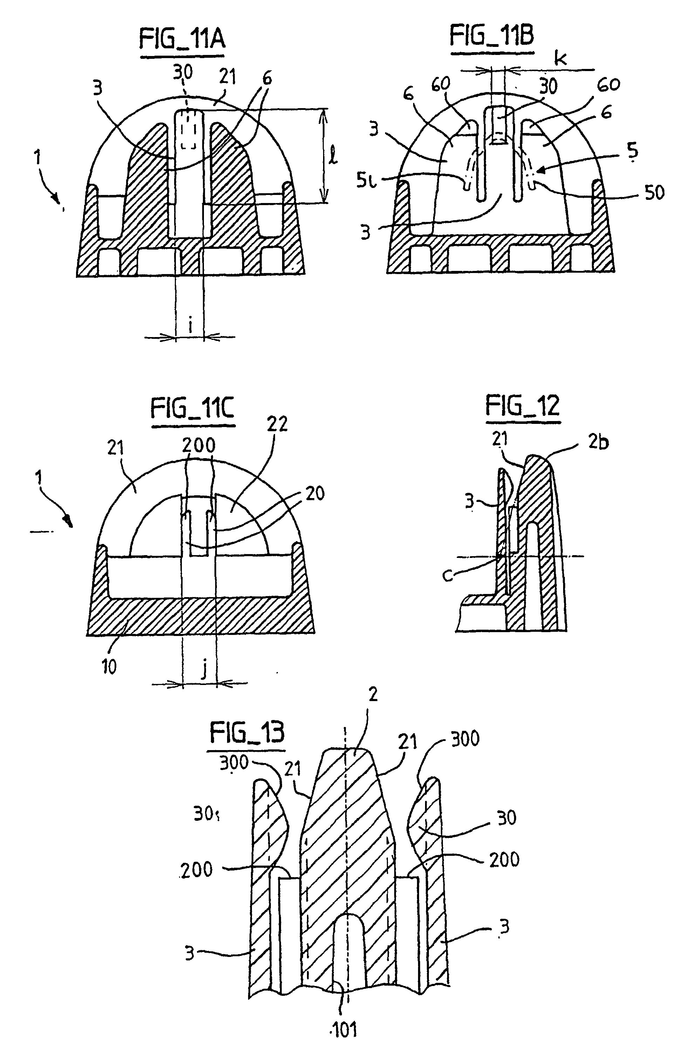

- les figures 11A, 11B et 11C sont des vues en coupe suivant les plans transversaux A-A, B-B (plan brisé) et, respectivement C-C (plan brisé) de la figure 9 ;
- la figure 12 est un détail de la figure 9, représentant une extrémité du support ;
- la figure 13 est un détail, à grande échelle, montrant la partie cerclée repérée XIII à la figure 9.

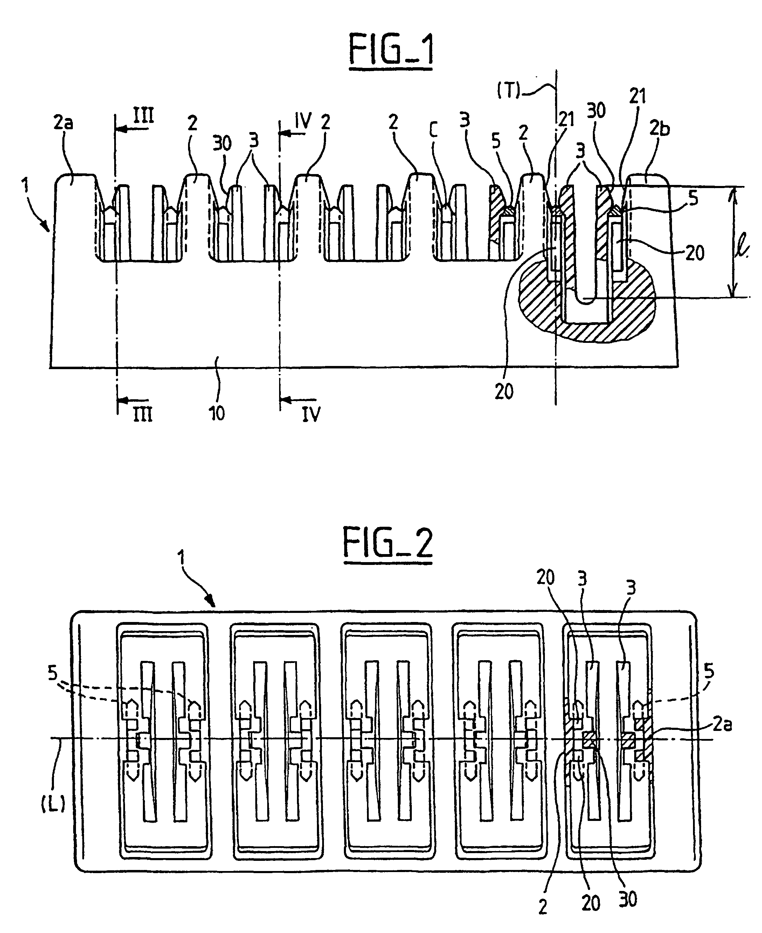

Le support de clips hémostatiques représenté sur les figures 1 à 4 est un bloc 1 en matière plastique possédant une certaine élasticité. Il s'agit par exemple d'un polycarbonate, moulé par injection. Ce bloc a la forme générale d'un parallélépipède rectangle dont la partie basse 10 est massive, et possède une face inférieure plane, destinée à être placée sur une table ou tout autre support horizontal.

Le support de clip est normalement saisi par ses extrémités, entre le pouce et l'index. Pour rendre plus sûre et confortable la préhension de la barrette, les extrémités de celle-ci présentent avantageusement des surfaces concaves et légèrement striées, ergonomiques, réduisant le risque de glissement accidentel entre la barrette et le gant chirurgical pouvant être humide.

La partie supérieure du bloc présente des aménagements permettant de recevoir côte-à-côte un ensemble de clips hémostatiques 5, représentés en traits interrompus sur les figures 1 à 4.

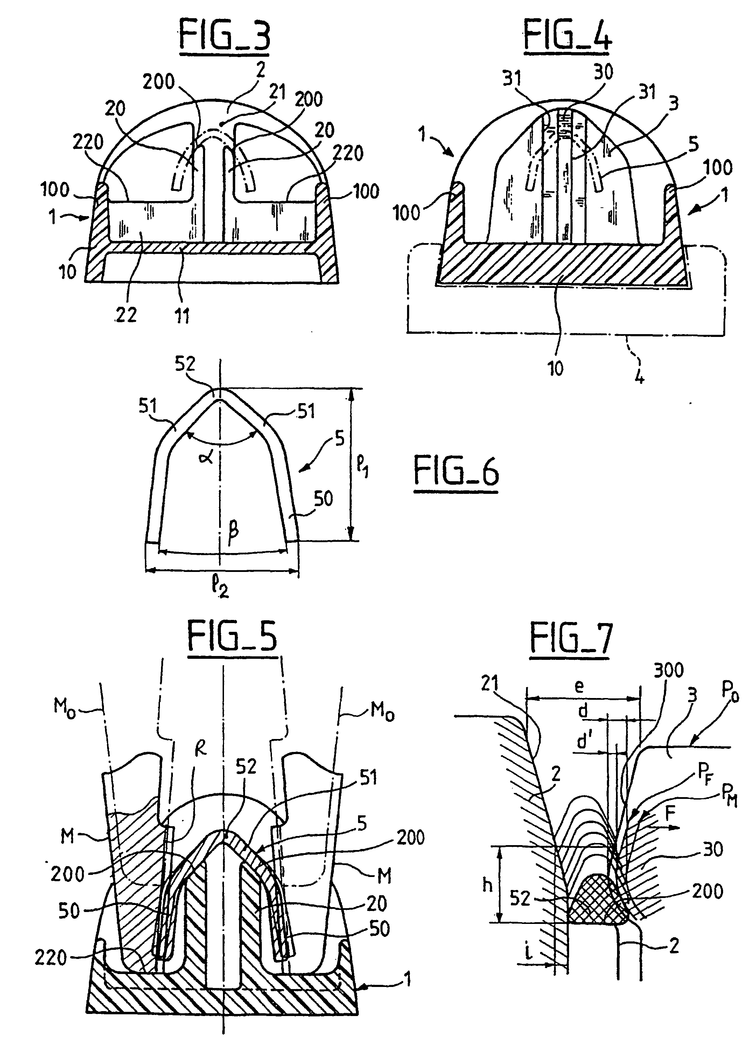

Comme le montre la figure 6, le clip 5 a la forme générale d'un "U" renversé qui comporte deux branches latérales 50 et une partie de fond 51, 52.

Cette partie de fond a la forme générale d'un "V" dont la zone centrale est référencée 52, tandis que les branches sont référencées 51.

L'angle de ce "V", référencé α, a par exemple une valeur de 90°.

Les branches 50 divergent également, mais très légèrement, d'un angle β de l'ordre de 15°.

Diverses dimensions de clips peuvent naturellement être prévues, en fonction des applications.

A titre indicatif, un "grand" clip pourra par exemple avoir une hauteur **l**_{**1**} de l'ordre de 11 mm et une largeur **l**_{**2**} de l'ordre de 10 mm.

Pour un "petit" clip, **l**_{**1**} et **l**_{**2**} pourront avoir une valeur de l'ordre de 3 à 4 mm.

Le clip est réalisé dans un fil métallique, de préférence en titane, ayant une section en forme de triangle approximativement équilatéral, mais à angles arrondis, la dimension de la section (côté du triangle) étant par exemple comprise entre 0,2 et 1 mm.

Il va de soi que les dimensions du support seront adaptées à ceux des clips qu'il doit contenir.

A titre indicatif, si on a affaire à un petit clip, le support 1 a par exemple une longueur de 28 mm, une largeur de 12 mm, et une hauteur de 9 mm.

Il est adapté pour recevoir dix clips côte-à-côte, en position retournée (ouverture du "U" tournée vers le bas), le plan de chaque clip s'inscrivant dans un plan vertical transversal, référencé **T** sur la figure 1.

Chaque clip 5 est emprisonné entre une partie fixe 2 et une partie élastiquement déformable 3.

Ces deux parties forment parties intégrantes de la partie basse 10, l'ensemble venant de moulage.

Les parties 2 et 3 affectent la forme générale de plaquettes transversales, les plaquettes 2 relativement épaisses étant indéformables, tandis qu'au contraire les plaquettes 3, plus minces, sont des languettes déformables élastiquement, par flexion, en direction longitudinale (perpendiculairement aux plans transversaux **T**).

Elles peuvent fléchir par rapport à leur zone de raccordement à la partie 10 du support, le matériau plastique constitutif de celui-ci ayant des propriétés élastiques choisies pour autoriser cette flexion.

La longeur **I** correspondant à la zone flexible des languettes 3 est, selon une caractéristique importante de l'invention, au moins égale - et de préférence supérieure - à la hauteur **I**_{**1**} des clips.

En référence aux figures 3 et 4, on notera la présence de nervures longitudinales latérales 100 qui rigidifient le bloc 1, de telle sorte que la force de préhension appliquée par l'opérateur ne perturbe pas l'extraction des clips. De plus, ces nervures constituent une ceinture de protection des clips.

A l'exception des parties d'extrémité fixes 2a et 2b, chaque partie fixe 2 coopère par ses deux faces opposées, avec une partie déformable 3, l'ensemble ayant une configuration "tête-bêche" qui se comprend à la simple observation des figures 1 et 2.

La face des parties 2 qui est en regard d'une partie déformable 3 présente deux nervures verticales parallèles 20 (voir figure 3) dont les bords supérieurs biseautés 200 sont conformés pour servir d'appui aux parties 51 du clip.

A un niveau supérieur à celui des faces d'appui 200, chaque partie 2 présente une facette 21 destinée à venir porter contre le flanc du clip, au niveau de sa zone centrale 52.

Comme on le voit plus particulièrement sur la figure 7, cette facette 21 est inclinée, de manière à se rapprocher vers la partie mobile 3 à laquelle elle est associée, lorsqu'on se dirige du haut vers le bas.

Chaque partie déformable 3 présente, sur sa face tournée vers la partie fixe associée 2, une patte de retenue bombée 30.

Celle-ci est conformée et positionnée de manière à venir s'appliquer convenablement contre le dessus de la zone 52 du clip, sur le flanc opposé à celui qui porte contre la facette 21.

Comme on le voit sur la figure 2, la patte 30 a une position axiale médiane par rapport aux deux nervures 20.

En regard de ces parties 20, la partie 3 est évidée et présente deux rainures verticales 31.

Comme on le voit sur la figure 7, la patte bombée 30 présente une face d'entrée inclinée 300, suivant une disposition sensiblement symétrique (par rapport à un plan transversal) de celle de la facette fixe 21. Sur cette figure, on distingue le contour triangulaire, à coins arrondis, de la section du clip, et plus particulièrement de la section de sa zone centrale 52.

La forme du triangle est sensiblement équilatérale, l'un des côtés étant horizontal. On comprend que lors de la mise en place du clip sur le support - opération qui se fait en usine - la partie 52 va être guidée entre les faces inclinées 21 et 30 au fur et à mesure de son enfoncement de haut en bas.

La facette fixe 21 joue le rôle d'une rampe, qui repousse axialement l'agrafe, laquelle repousse à son tour la patte élastique 30 (flèche **F**, figure 7).

Une fois que la base de la section de zone 52 a passé la partie bombée de la patte 30, celle-ci peut se déformer en sens inverse de **F**, sur une course néanmoins limitée, de manière à venir presser contre la zone 52, pour l'appliquer élastiquement à la fois sur les faces d'appui 200 et contre la base de la facette 21.

La patte élastique 30 exerce donc une force dirigée à la fois axialement, vers l'élément fixe 2, et vers le bas, par suite de la forme triangulaire de la section du clip.

Le clip se trouve ainsi parfaitement immobilisé dans le support au niveau de la zone 52 et de ses branches intermédiaires 51.

En revanche, ses branches 50 sont complètement libres, et accessibles de telle sorte que son extraction au moyen d'une pince peut se faire très commodément.

Sur le schéma de la figure 7 on a désigné par **P**_{**0**} le contour de la partie flexible 3, avant mise en place d'un clip, par **P**_{**M**} la position d'écartement maximal en cours de mise en place du clip, et par **P**_{**F**} la position finale, dans laquelle la patte 30 immobilise le clip sur son support.

A titre indicatif, on indiquera ci-après quelques dimensions fonctionnelles référencées sur le schéma de la figure 7 :
- écartement à l'entrée entre les facettes 21 et 300 : **e** = 2 mm ;
- course de recul maximal de la patte mobile : **d** = 0,33 mm ;
- course de recul de la patte 30 en position d'immobilisation du clip : **d**' = 0,15 mm ;
- course de descente du clip qui provoque le recul de la patte mobile 30 : **h** = 1,35 mm ;
- translation axiale du clip au moment de cet enfoncement : **i** = 0,24 mm.

La figure 5 illustre le prélèvement et l'extraction d'un clip au moyen d'une pince dont les deux mors - ou mâchoires - sont référencés **M**.

On a représenté en traits interrompus mixtes fins, et référencé **M**_{**0**}, les mâchoires de la pince avant leur engagement avec le clip.

Ces mâchoires comportent, de manière bien connue, des rainures longitudinales débouchantes **R** (ouvertes vers le bas), qui viennent enserrer le bord externe des branches 50.

L'une de ces rainures est visible sur la mâchoire de gauche, représentée en coupe, à la figure 5.

On comprend à la simple observation de la figure 5 que la descente des mâchoires se fait librement, aucun élément du support 1 n'étant présent dans la zone d'action des mâchoires **M.**

Les mâchoires réalisent un certain pincement du clip au cours de leur descente (fermeture du "U") de manière à le retenir par friction.

La course de descente de la pince est limitée par des faces horizontales 220 formant butée, qui s'étendent vers l'extérieur à partir des éléments 20.

L'opérateur n'a donc pas à tâtonner. En fin de course, le clip est correctement en place dans la pince.

Il ne lui reste plus alors qu'à exercer une traction verticale, dans le plan **T** du clip, du bas vers le haut.

Le désengagement du clip s'obtient par effet de rampe, dans une direction inverse par rapport à celle de sa mise en place décrite précédemment en référence à la figure 7.

La partie 52 du clip, qui frotte contre la paroi bombée de la patte 30, provoque l'ecartement de celle-ci suivant la flèche **F**, et le clip se trouve rapidement libéré.

Il est en principe possible de prélever le clip d'une seule main au moyen de la pince, l'effort nécessaire à l'enlèvement du clip étant faible et régulier, pratiquement constant (sans point dur), insuffisant pour soulever le support

Cependant, il peut être utile, comme cela est connu déjà, d'emboîter le support dans un socle lourd.

Cette possibilité est illustrée à la figure 4, sur laquelle le contour d'un socle lourd 4 a été dessiné en traits interrompus.

A cet effet, la base 10 du support a avantageusement des faces latérales inclinées, formant queue d'aronde, apte à s'emboîter dans une rainure (glissière) de forme complémentaire prévue dans le socle.

Une autre solution peut consister à coller contre la face inférieure du support 1 un film adhésif double face. Au moment de l'intervention, l'opérateur peut ainsi le coller sur un support approprié, par exemple sur l'un de ses gants.

Dans une variante possible (non illustrée) du support, l'écartement de la patte 30 (dans le sens **F**) pourrait être obtenu par action de la pince au moment de son introduction dans le support. Il suffit pour cela de prévoir des rampes de forme appropriée sur la partie élastique 3, contre laquelle viennent porter les zones latérales des mors de la pince au cours de leur descente, libérant alors le clip. L'effort nécessaire à l'extraction se trouve ainsi diminué.

Le support qui fait l'objet de la variante des figures 8 à 13 est particulièrement adapté à des clips de moyenne ou petite dimension. Il est adapté pour recevoir six clips.

Sur ces figures, les mêmes chiffres de références ont été utilisées pour désigner les éléments identiques ou similaires à ceux du support qui vient d'être décrit.

On notera que le support 1 présente, dans sa partie basse, des espaces d'allègement 101, dont certains s'étendent à l'intérieur des parties fixes 2. On notera aussi la forme concave des extrémités 102 du support, qui en facilitent la prise en main.

Comme on le voit plus particulièrement sur les figures 9, 11a et 11B, la languette mince et élastique 3 est de faible largeur **i** ; elle occupe uniquement la partie centrale du support. Son épaisseur (visible sur la figure 9) diminue du bas vers le haut et lui confère une section transversale triangulaire ; ainsi, la contrainte de flexion est sensiblement constante sur toute sa hauteur.

Cette hauteur est supérieure à la hauteur du clip, comme le montre la figure 11B où un clip (en place sur le support) a été représenté en traits interrompus.

La patte bombée 30 occupe la zone centrale de la partie haute de la languette. Sa largeur **k** est par exemple moitié de la largeur **i**.

A la figure 11C, on a désigné par **j** la largeur des éléments d'appui 20.

Cette largeur est sensiblement plus grande que la largeur **i** ci-dessus mentionnée.

La languette 3 est bordée, sur chacun de ses bords, d'un élément fixe 6 se dressant vers le haut. Le bord supérieur 60 des éléments 6 est légèrement chanfreiné.

Les éléments 6 ont la forme de piliers reliés à leur base à la partie basse de l'élément 1.

Les faces transversales des éléments 6 qui sont tournées vers la partie fixe associée 2, se trouvent sensiblement dans le même plan que la face correspondante de la languette 3.

Les branches latérales libres 50 du clip se trouvent par conséquent entre deux faces fixes, si bien que la mise en place de la pince en vue de son prélèvement ne risque pas de solliciter intempestivement la languette élastique. Elle est sollicitée seulement au cours de l'extraction subséquente, après que les rainures de la pince aient été correctement engagées sur les branches 50. Les bords biseautés 60 facilitent le guidage de la pince.

Les éléments 6 (et les chanfreins 60) ont une hauteur telle que les mors de la pince sont guidés avant même que le contact ne se produise entre les mors et le clip.

On notera que la rampe fixe 21 a une forme tronconique, dont le demi-angle au sommet a par exemple une valeur de 75°, et dont le sommet (fictif) désigné C sur la figure 12, se situe dans la zone centrale de la languette 3 associée.

Cette partie tronconique est coupée par des faces verticales transversales 22 de chaque côté de la languette (voir figure 11C).

A titre indicatif, pour un support destiné à recevoir des clips dont la hauteur **l**_{**1**} est de l'ordre de 4 à 5 mm, on indiquera les valeurs dimensionnelles suivantes : **l** = 8 à 10 mm ; **j** = 2,5 à 3,5 mm ; **i** = 2 mm ; **k** = 1 mm.

## Revendications

1. Support de clips hémostatiques, pour un ensemble de clips (5) ayant approximativement la forme d'un "U" comprenant une partie de fond (51-52) et une paire de branches latérales (50), ce support (1) consistant en une barrette allongée présentant des moyens pour supporter et retenir individuellement les clips côte-à-côte en position renversée, dans des plans parallèles transversaux (T), dans lequel ces moyens coopèrent exclusivement avec les parties de fond (51-52) du clip (5), tandis que ses branches latérales (50) sont libres, **caractérisé par le fait que** lesdits moyens de support et de retenue de chacun des clips (5) consistent, d'une part en une partie fixe (2) qui porte au moins une face d'appui (200) sur laquelle vient reposer la partie de fond (51-52) du clip (5), d'autre part en une partie mobile (3) en forme de languette mince élastiquement déformable par flexion, dont la longueur (1) est au moins égale - et de préférence supérieure - à la hauteur (1₁) du clip, et qui comporte en partie haute une patte de blocage (30) à paroi convexe (bombée) venant s'appliquer contre la zone centrale (52) de ladite partie de fond du clip pour la retenir, ces deux parties (2, 3) présentant des parois d'entrée (21, 300) convergentes qui assurent le guidage du clip (5) et l'écartement provisoire de la patte de blocage (30) au moment de sa mise en place.

2. Support selon la revendication 1, **caractérisé par le fait que** la partie fixe (2) possède deux faces d'appui (200) qui supportent les zones intermédiaires (51) du clip. (5) situées entre la zone centrale (52) et les branches latérales (50).

3. Support selon l'une des revendications 1 ou 2, **caractérisé par le fait que** la partie fixe (2) possède une face (220) formant butée de fin de course pour la pince de prélèvement des clips.

4. Support selon l'une des revendications 1 à 3, **caractérisé par le fait que** les différentes parties fixes (2) et déformables (3) sont placées tête-bêche, deux à deux, de sorte que chaque partie fixe (2) - à l'exception des parties d'extrémité (2a, 2b) - assure la retenue de deux clips (5), en association avec deux pattes déformables (30).

5. Support selon l'une des revendications 1 à 4, **caractérisé par le fait que** ladite partie mobile (3) en forme de languette, possède une largeur (i) sensiblement inférieure à celle du clip (5), et qu'elle est bordée d'éléments fixes (6) en regard desquels viennent se placer les branches latérales (50) du clip.

## Claims

1. Haemostatic clip holder for a set of approximately "U"-shaped clips (5) comprising a bottom part (51-52) and a pair of side arms (50), this holder (1) consisting of an elongated cartridge provided with means of supporting and individually retaining clips side by side in the upside down position, in parallel transverse planes (T), in which these means cooperate exclusively with bottom parts (51-52) of the clip (5), whereas its side arms (50) are free, **characterized by** the fact that the said support and retaining means for each of the clips (5) consist firstly of a fixed part (2) that supports at least one support surface (200) on which the bottom part (51-52) of the clip (5) rests, and secondly a mobile part (3) in the form of a thin strip elastically deformable by bending, the length (1) of which is at least equal to (and preferably greater than) the height (I₁) of the clip and which includes a blocking tab (30) with a convex surface (curved) in its upper part that comes into contact with the middle part (52) of the said part of the bottom of the clip to retain it, these two parts (2, 3) having tapered entry walls (21, 300) to guide the clip (5) and control temporary separation of the blocking tab (30) at the time that it is positioned.

2. Holder according to claim 1, **characterized by** the fact that the fixed part (2) has two support surfaces (200) that support intermediate areas (51) of the clip (5) located between the middle part (52) and the side arms (50).

3. Support according to any one of claims 1 or 2, **characterized by** the fact that the fixed part (2) has a face (220) that forms a limit stop for the clip gripping pliers.

4. Holder according to any one of claims 1 to 3, **characterized by** the fact that the various fixed parts (2) and deformable parts (3) are placed head to foot in pairs, such that each fixed part (2) except for the end parts (2a, 2b) retains the two clips (5), working in cooperation with two deformable tabs (30).

5. Holder according to any one of claims 1 to 4, **characterized by** the fact that the width (i) of the said mobile part (3) in the form of a strip is significantly less than the width of the clip (5), and that it is surrounded by fixed elements (6) adjacent to the positions of the side arms (50) of the clip.

## Patentansprüche

1. Träger für hämostatische Klammem für eine Klammeranordnung (5), welche näherungsweise die Form eines "U" mit einem Bodenbereich (51-52) und einem Paar Seitenschenkeln (50) aufweist, wobei dieser Träger (1) aus einem langgestreckten Balken besteht, der Vorrichtungen aufweist, um die Klammern einzeln nebeneinander in umgekehrter Position in parallelen Querebenen (T) zu tragen und zu halten, wobei diese Vorrichtungen ausschließlich mit den Bodenbereichen (51-52) der Klammem zusammenwirken, während ihre Seitenschenkel (50) frei sind, **dadurch gekennzeichnet, dass** die Vorrichtungen zum Tragen und zum Halten jeder der Klammern (5) einerseits aus einem festen Bereich (2), der wenigstens eine Auflageflache (200) trägt, auf welcher der Bodenbereich (51-52) der Klammer zu ruhen kommt, und andererseits aus einem beweglichen Bereich (3) in Form einer durch Biegung elastisch verformbaren, dünnen Lasche bestehen, deren Länge (I) wenigstens gleich - und vorzugsweise länger - als die Höhe (Iᵢ) der Klammer ist, und welche im oberen Bereich eine Verriegelungsklemme (30) mit konvexer Wand (bauchig) aufweist, die sich gegen den mittleren Bereich (52) des Bodenbereiches der Klammer presst, um sie zu halten, wobei diese beiden Bereiche (2, 3) konvergierende Eingangswände (21, 300) aufweisen, welche die Führung der Klammer (5) und die provisorische Spreizung der Verriegelungsklemme (30) zum Zeitpunkt deren Anordnung sicherstellen.

2. Träger gemäß Anspruch 1, **dadurch gekennzeichnet, dass** der feste Bereich (2) zwei Auflageflächen (200) besitzt, welche die Zwischenbereiche (51) der Klammer (5), die zwischen dem Mittelbereich (52) und den Seitenschenkeln (50) angeordnet sind, tragen.

3. Träger gemäß einem der Ansprüche 1 oder 2, **dadurch gekennzeichnet, dass** der feste Bereich (2) eine Fläche (220) aufweist, die einen Laufendanschlag für die Greifzange der Klammem bildet

4. Träger gemäß einem der Ansprüche 1 bis 3, **dadurch gekennzeichnet, dass** die verschiedenen festen (2) und verformbaren Bereiche (3) paarweise, zueinander umgedreht angeordnet sind, so dass jeder feste Bereich (2) - mit Ausnahme der Endbereiche (2a, 2b) - das Halten von zwei Klammem (5) in Verbindung mit zwei verformbaren Klemmen (30) sicherstellt.

5. Träger gemäß einem der Ansprüche 1 bis 4, **dadurch gekennzeichnet, dass** der bewegliche Bereich (3) in Form einer Lasche eine Breite (i) besitzt, die praktisch kleiner als diejenige der Klammer (5) ist, und dass er von festen Elementen (6) gesäumt ist, gegenüber denen sich die Seitenschenkel (50) der Klammer anordnen.
